# EUROPEAN PATENT APPLICATION

(11) **EP 2 108 317 A2**
(43) Date of publication of application: **14.10.2009**
(21) Application number: 09004584.0
(22) Date of filing: 30.03.2009
(51) Int. Cl.: A61B 17/04

(54) **Suture device**

(30) Priority: 11.04.2008 US 101320
(71) Applicant: Olympus Medical Systems Corporation, Tokyo 151-0072 (JP)
(72) Inventor: Shiono, Junji, Tokyo 151-0072 (JP); Hayashi, Kensuke, Tokyo 151-0072 (JP); Suzuki, Takayuki, Tokyo 151-0072 (JP)
(74) Representative: von Hellfeld, Axel

(57) **Abstract**

A suture device (1) which sutures tissue using a suture unit having a first anchor (106A, 106C) and a second anchor (106B) respectively attached to both ends of a suture thread (104), wherein a hollow distal member (4) is provided which houses said first anchor and second anchor, the end face of the distal side of said distal member is formed so as to constitute a first sharp angle with the axis of said distal member, the distal side of said second anchor is provided with a sloped face (109B) so as to constitute a second sharp angle with the axis of said second anchor which is an angle at or below said first sharp angle, and said first anchor and said second anchor are axially aligned and housed inside said distal member so that said second anchor is positioned on the proximal side.

## Description

### BACKGROUND OF THE INVENTION

### Field of the Invention

The present invention relates to a suture device that is inserted into a body cavity for use, and more particularly to a suture device employed when suturing perforations and the like which have formed in hollow organs such as the stomach, intestine or the like using suture thread which has anchors attached to both ends.

Priority is claimed on United States Patent Application No.12/101,320, filed on April 11, 2008, the content of which is incorporated herein by reference.

### Description of Related Art

Heretofore, suture devices have been known which use suture thread having anchors attached to both ends for the purpose of suturing perforations, lacerations and the like which have formed in hollow organs such as the stomach, intestine or the like (e.g., see International Unexamined Patent Application, First Publication No. 2007-37326). With this suture device, suturing is conducted by having the anchors at both ends of the suture thread engage with tissue near the perforation at the interior or exterior of the tissue, and by drawing together the tissue engaged by the anchors by tightly pulling the suture thread.

In order to reliably conduct suturing by the suture device recorded in International Unexamined Patent Application, First Publication No. 2007-37326, the anchors loaded in the distal end of the suture device must be reliably ejected one at a time from the distal end into the tissue opposite the periphery of the perforation or the like, and must engage with the tissue.

However, with respect to the suture device recorded in International Unexamined Patent Application, First Publication No. 2007-37326, when ejecting the anchors, it may happen that the rear end of the anchor catches on the edge of the needle, and is not able to be smoothly ejected.

When the first anchor is fully pushed out so that its rear end does not catch on the edge of needle, a portion of the second anchor is then exposed from the end face of the needle, with the result that puncturing for ejection of the second anchor may become difficult.

### SUMMARY OF THE INVENTION

The present invention has been made in light of the foregoing circumstances, and its object is to offer an endoscopic treatment tool capable of smoothly ejecting anchors from the distal end.

The first aspect of the present invention is a suture device which sutures tissue using a suture unit having a first anchor and a second anchor respectively attached to the two ends of a suture thread, wherein a hollow distal member is provided which houses said first anchor and second anchor, the end face of the distal side of said distal member is formed so as to constitute a first sharp angle with the axis of said distal member, the distal side of said second anchor is provided with a sloped face so as to constitute a second sharp angle with the axis of said second anchor which is an angle at or below said first sharp angle, and said first anchor and said second anchor are axially aligned and housed inside said distal member so that said second anchor is positioned on the proximal side.

### BRIEF DESCRIPTION OF THE DRAWINGS

FIG. 1 is a figure which shows the suture device of one embodiment of the present invention.
FIG. 2 is an enlarged view which shows a partial section of the distal end of this suture device.
FIG. 3 is a figure which shows the suture unit used in this suture device.
FIG. 4 is an oblique view which shows the needle and suture unit of this suture device.
FIG. 5 is a frontal view which shows this needle and this suture unit.
FIG. 6 is a figure which shows a partial section of the operating portion of this suture device.
FIG. 7 is a figure which shows operations during use of this suture device.
FIG. 8 is a figure which shows conditions pertaining to the insertion of this suture device into target tissue.
FIG. 9A and FIG. 9B are enlarged views which respectively show partial sections of the distal-end operating portion during use of this suture device, and the distal end during use of this suture device.
FIG. 10 is a figure which shows one process of suturing by this suture device.
FIG. 11A and FIG. 11B are enlarged views which respectively show partial sections of the distal-end operating portion during use of this suture device, and the distal end portion during use of this suture device.
FIG. 12 is a figure which shows one process of suturing by this suture device.
FIG. 13 is a figure which shows one process of suturing by this suture device.
FIG 14 is a frontal view which shows a needle and suture unit pertaining to a modified example of this suture device.
FIG. 15A is a figure which shows an anchor of the suture unit housed in a needle pertaining to a modified example of this suture device.
FIG. 15B is a figure which shows an anchor of the suture unit housed in the needle of the suture device of one embodiment of the present invention.

### DETAILED DESCRIPTION OF THE INVENTION

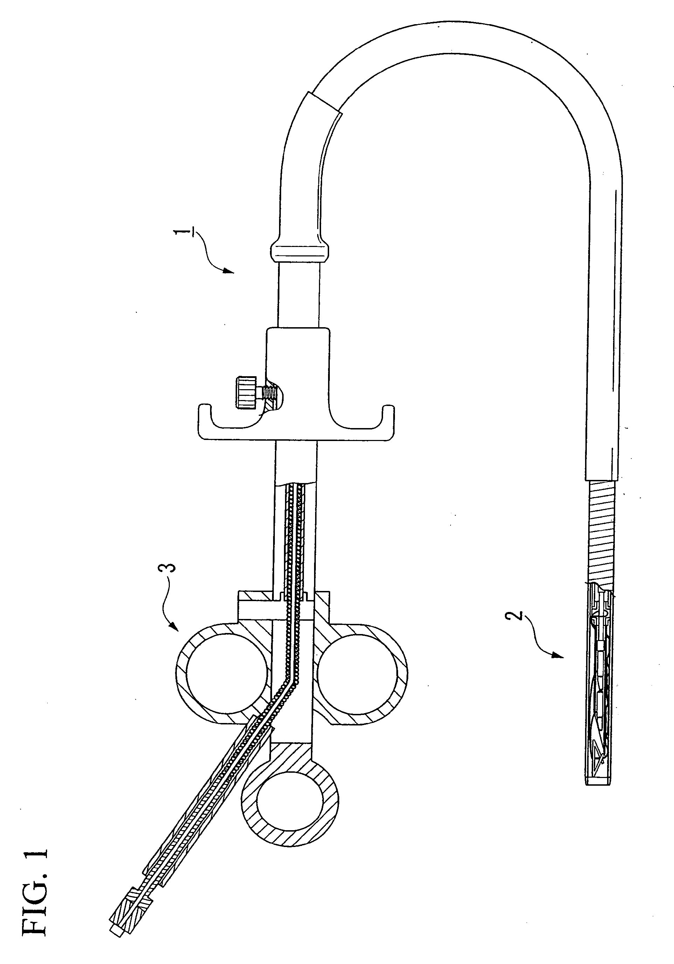

Hereinbelow, the suture device of one embodiment of the present invention shall be described with reference to FIG 1 to FIG. 15B.

FIG. 1 is a figure which shows a suture device 1 of this embodiment. As shown in FIG. 1, the suture device 1 is provided and configured with a distal end portion 2 inserted into the body and an operating portion 3 for manipulating the respective mechanisms of the distal end portion 2.

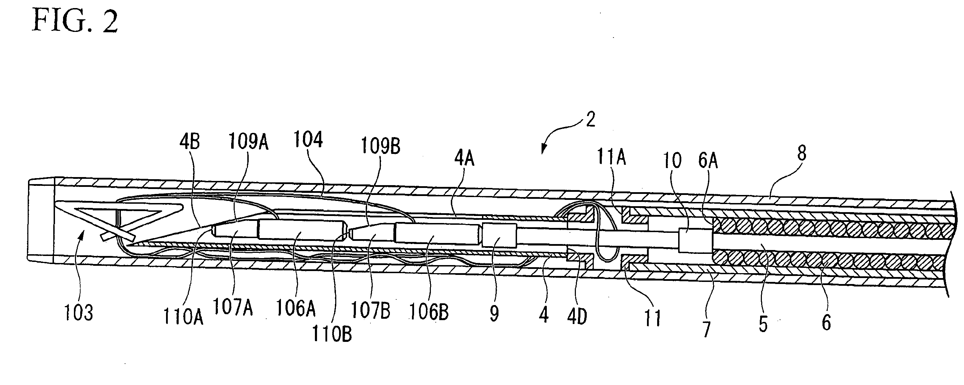

FIG. 2 is an enlarged view which shows a partial section of the distal end portion 2. The distal end portion 2 is provided and configured with a needle (distal member) 4 to which the below-mentioned suture unit is attached, a wire 5 which runs through the needle 4, a second sheath (parallel member) 6 through which runs the proximal end of the wire 5, a tube 7 through which the wire 5 and second sheath 6 are inserted in the axial direction with free retraction and advancement, and which is integrally fixed to the needle 4 on the proximal side of the needle 4, and a first sheath (sheath) 8 through which the tube 7 is inserted.

The needle 4 is a hollow member formed by metal or the like, and is a so-called beveled needle which is diagonally cut so that its tip constitutes a sharp angle with the axis. A groove 4A is formed on the top face of the needle 4. The anchors of the suture unit are housed inside the needle 4.

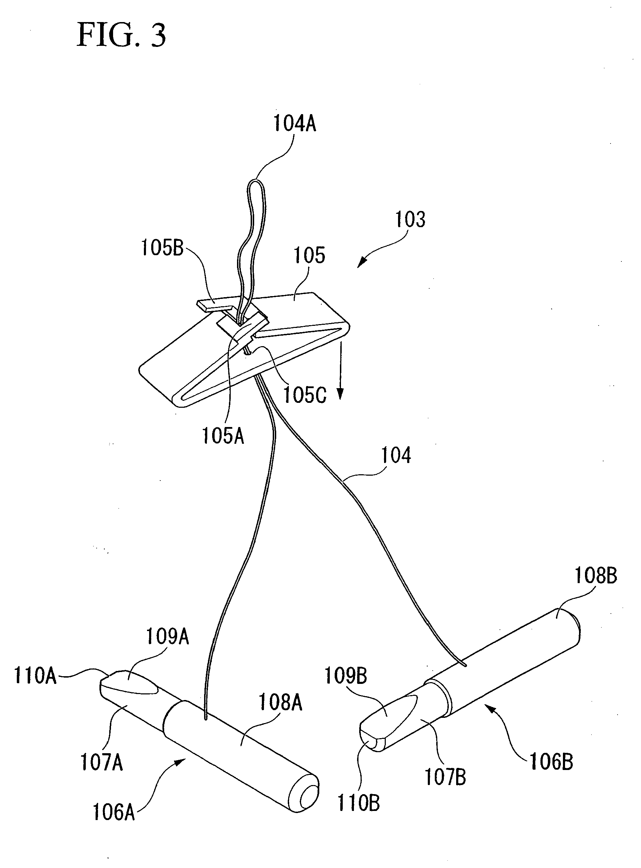

FIG. 3 is a figure which shows a suture unit 103 housed in the needle 4. The suture unit 103 is provided and configured with suture thread 104, a stopper 105 through which the suture thread 104 runs, and a rod-like first anchor 106A and second anchor 106B attached to the two ends of the suture thread 104.

The stopper 105 is a plate-like member composed of metal or resin such as biodegradable resin or the like, and is bent so that left and right end portions 105A and 105B face opposite each other, and is formed so that the end portions 105A and 105B mutually engage.

A hole 105C is provided near the center in the lateral direction of the stopper 105, and the suture thread 104 folded at center point 104A runs through the hole 105C from the face on the opposite side of the end portions 105A and 105B, and is disposed so that it passes between portion 105A and portion 105B which mutually engage. Operations during use of the stopper 105 are described below.

The first anchor 106A and second anchor 106B are members which are approximately shaped like round bars, and connect to the suture thread 104 at a position near the center in the lengthwise direction. Each anchor 106A and 106B respectively has a small-diameter portion 107A and 107B on the distal side, and a large-diameter portion 108A and 108B on the proximal side. Moreover, the respective small-diameter portions 107A and 107B of the anchors 106A and 106B are cut so that a sharp angle is constituted relative to the axis, thereby forming sloped faces 109 A and 109B.

It is preferable that the angles (second sharp angles) constituted relative to the axis of each anchor 106A and 106B by the sloped faces 109A and 109B be set at or below the angles (first angles) constituted by end face 4B of the distal side of the needle 4 relative to the axis of the needle 4. With respect to the suture device 1 of the present embodiment, the angle constituted by end face 4B and the axis of the needle 4 is 15 degrees, while the angles constituted by the sloped faces 109A and 109B relative to the respective axis of the anchors 106A and 106B are set at 12-13 degrees.

Furthermore, each sloped face 109A and 109B is cut orthogonally relative to the respective axis of the anchors 106A and 106B, thereby forming each distal end face 110A and 110B.

As shown in FIG. 2, the first anchor 106A and second anchor 106B of the suture unit 103 are housed inside the needle 4 in a condition where they are axially aligned with the first anchor 106A placed on the distal side. The suture thread 104 connected to each anchor 106A and 106B is exposed to the exterior of the needle 4 from the groove 4 A.

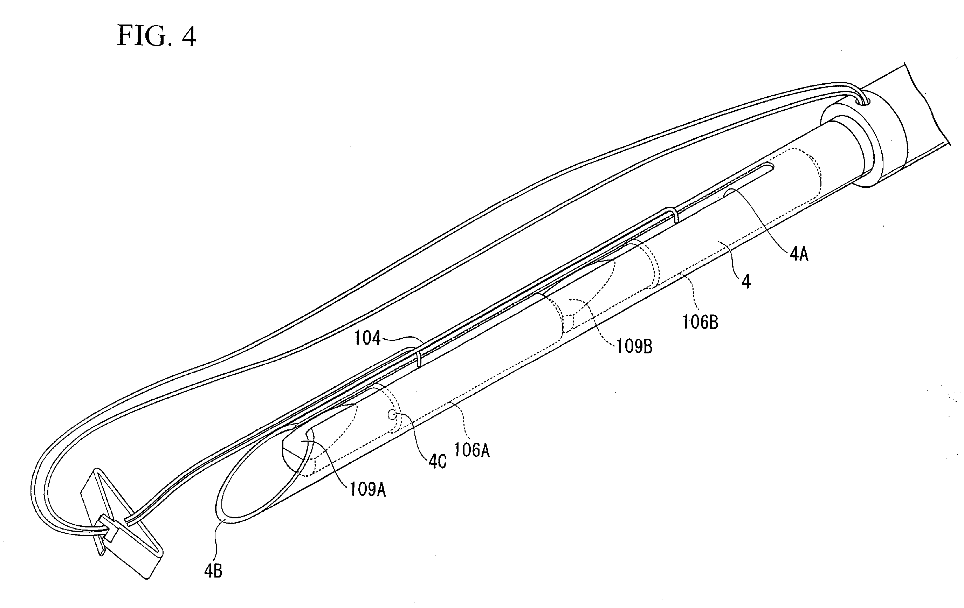

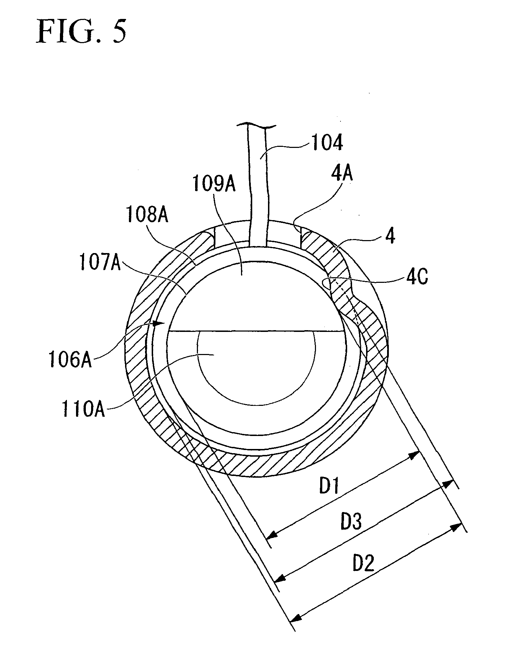

As shown in FIG. 3, the suture thread 104 is connected to the outer circumferential face of each anchor 106A and 106B, at positions where the phases in the circumferential direction relative to the rear ends of the sloped faces 109A and 109B are approximately identical. Consequently, as shown in FIG. 4 and FIG. 5, when each anchor 106A and 106B is housed inside the needle 4 in a state where the suture thread 104 is drawn out from the groove 4A, the end face 4B of the needle 4 and the sloped faces 109A and 109B have approximately identical orientations. In this instance, when the thickness of the suture thread 104 and the width of the groove 4A are set so as to be approximately identical, the alignment of the end face 4B and sloped faces 109A and 109B is more satisfactory.

Furthermore, as shown hi FIG. 4 and FIG. 5, a portion of the outer circumferential face of the needle 4 is formed so that is projects into the cavity, and a projection 4C is provided. As shown in FIG. 5, diameter D1 of the small-diameter portions 107A and 107B of each anchor 106 A and 106B is smaller than diameter D2 of the inscribed circle in the cavity of the needle 4 involving the projection 4C, and diameter D3 of the large-diameter portions 108A and 108B is set larger than diameter D2. FIG. 5 only shows the first anchor 106A on the distal side, but the same also applies to the second anchor.

The wire 5 is formed from metal or the like, and as shown in FIG. 2, its distal end runs through the needle 4 from the proximal end 4B of the needle 4. A pressure member 9 is attached to the distal end portion of the wire 5. It causes the wire 5 to advance toward the distal side of the needle 4 in the axial direction, enabling the first anchor 106A and second anchor 106B to be pressed and ejected to the outside of the needle 4.

As the wire 5, it is preferable to have a single wire capable of satisfactorily transmitting the pressing force imparted by the operating portion 3 to the pressure member 9, but it is also possible to apply multiline wires which intertwine metal wires, or coiled wire formed by winding metal wires or multiline wires into a coil, or the like.

At a position separated by a prescribed distance from the pressure member 9 of the wire 5, an annular contact member (relative position maintenance member) 10 is attached and fixed which uniformly maintains the relative positional relation of the wire 5 and second sheath 6. The dimensions of the outer diameter of the contact member 10 are set to a size which enables free retraction and advancement through the interior of the needle 4. The contact member 10 does not necessarily have to be annular if it radially projects toward the outside from the wire 5.

The second sheath 6 is a coil sheath in which metal wire or multiline wire is wound into a tubular form, and the proximal end of the wire 5 runs through it in a freely retractable manner in the axial direction. The inner diameter of the second sheath 6 is set smaller than the outer diameter of the contact member 10 of the wire 5, and the contact member 10 contacts the distal end portion 6A of the second sheath 6 so that it is unable to advance into the interior of the second sheath 6. That is, when the contact member 10 and distal end portion 6A of the second sheath 6 come into contact, the positional relation of the wire 5 and second sheath 6 is uniformly maintained.

The tube 7 is a flexible, annular member which is composed of resin or the like. As the material of the tube 7, it is preferable to have resin material or the like which does not stretch much in the axial direction. The tube 7 is integrally connected to the proximal end 4B of the needle 4 via a connecting tube (forward regulating member) 11 attached to its distal end.

On the outer face of the connecting tube 11, a through-hole (communicating portion) 11A is provided which penetrates to the cavity. The bent center point 104A of the suture thread 104 of the suture unit 103 is inserted into the cavity of the connecting tube 11 from the through-hole 11A and is wrapped around the wire 5 which runs through the interior.

The inner diameter in the axial direction of the connecting tube 11 is set larger than the outer diameter of the contact member 10 of the wire 5, and the contact member 10 is able to freely advance and retract through the interior of the connecting tube 11 in the axial direction. At the same time, the inner diameter of the connecting tube 11 in the axial direction is set smaller than the outer diameter of the second sheath 6, and is configured so that the second sheath 6 cannot enter into the connecting tube 11.

The first sheath 8 is a coil sheath with the same structure as the second sheath 6, and the tube 7 and the needle 4 integrally connected to the tube 7 run through it in the axial direction with free retraction and advancement. As shown in FIG 2, the entirety of the suture unit 103 loaded in the needle 4 can be housed in its cavity.

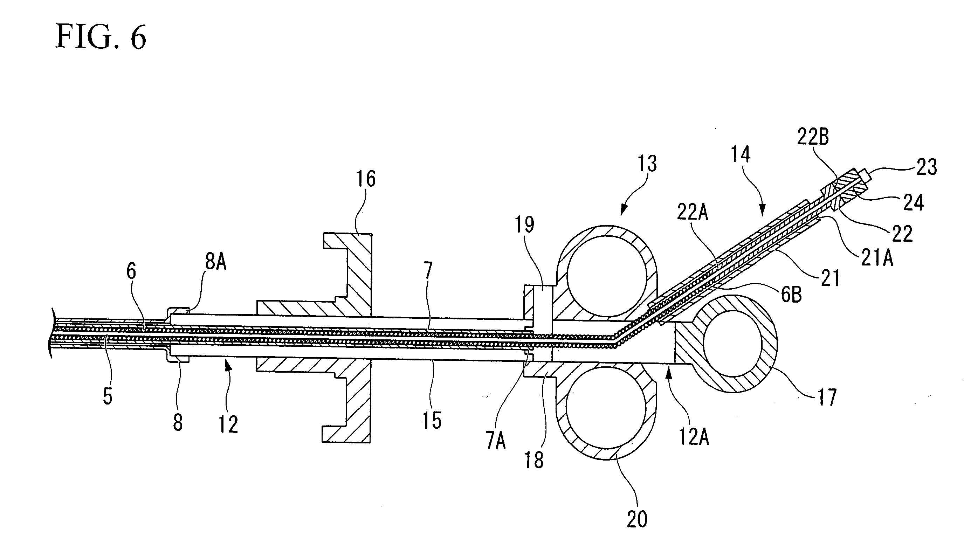

FIG. 6 is a figure which shows a partial section of the operating portion 3. The operating portion 3 provided on the proximal side of the wire 5 and second sheath 6 is provided and configured with a body 12 to which the proximal end 8A of the first sheath 8 is fixed, a sliding portion 13 attached to the body 12 so that it is able to slide in the axial direction of the body 12, and a distal-end operating portion 14 fixed to the sliding portion 13.

The body 12 is formed from resin or the like, and is configured by a pair of rod-like side wall members 15 which are aligned in parallel. The proximal end 8A of the first sheath 8 is fixed to the distal end portion of the body 12 by such means as adhesive bonding or caulking. An approximately cylindrical adjuster 16 made of resin or the like is attached near the distal end portion of the body 12 so as to surround the pair of side wall members 15.

The adjuster 16 is capable of sliding in the axial direction of the body 12, and is configured so that it may be fixed at an arbitrary position on the body 12 by a fixing means such as screws (not illustrated in the drawing). By changing the fixing position of the adjuster 16, it is possible to adjust the projection amount of the needle 4 from the first sheath 8 in the below-described manner.

At the proximal end 12A of the body 12, an annular finger catch 17 is provided which is integrated with the pair of side wall members 15.

The sliding portion 13 is provided and configured with a slider 18 which is attached to the body 12 so as to be capable of sliding, and a connecting member 19 fixed to the slider 18.

The slider 18 is an approximately tubular member composed of resin or the like which is attached to the proximal end 12A side of the body 12 by the adjustor 16 so as to surround the pair of side wall members 15. The slider 18 is capable of sliding in the axial direction of the body 12 between the adjustor 16 and finger catch 17. A handle 20 is provided in the slider 18 so that the user can engage his fingers during operation.

The connecting member 19 is composed of resin, metal or the like, and the proximal end 7A of the tube 7 which extends between the pair of side wall members 15 of the body 12 from the proximal end 8A of the first sheath 8 is fixed in place by a method such as welding or adhesive bonding. That is, the proximal end 7A of the tube 7 is fixed to the slider 18 via the connecting member 19, and it is possible to advance and retract the tube 7 within a certain range in the axial direction of the body 12 by sliding the slider 18.

The distal-end operating portion 14 is provided and configured with a tubular member 21 fixed to the slider 18, a sheath operating member 22 which runs through the tubular member 21, and a wire operating knob 23 attached to the proximal end of the wire 5.

The tubular member 21 is composed of resin or the like, and is fixed to the rear of the handle 20 of the slider 18. The wire 5 and second sheath 6 extending from the proximal end 7A of the tube 7 run through the tubular member 21.

The sheath operating member 22 is a tubular-shaped member composed of resin or the like, and is inserted into the tubular member 21 from the proximal end 21A side of the tubular member 21. The sheath operating member 22 is capable of sliding in the axial direction of the tubular member 21, and the proximal end 6B of the second sheath 6 is fixed to its distal end 22A by a means such as adhesive bonding or caulking. The wire 5 extending from the proximal end 6B of the second sheath 6 passes through the cavity of the sheath operating member 22, and is exposed from the rear end 22B of the sheath operating member 22.

The wire operating knob 23 is a disk-like member, and is attached to the proximal end of the wire 5 which is exposed from the rear end 22B of the sheath operating member 22. The wire operating knob 23 does not have to be like a disk, and it may assume any shape such as that of a round bar or square bar so long as it is a shape that engages with the sheath operating member 22 and the below-mentioned wire stopper.

A wire stopper 24 - which serves to uniformly maintain the positional relation of the wire 5 and second sheath 6 and to prevent malfunctions - is interposed between the rear end 22B of the sheath operating member 22 and the wire operating knob 23 in such a way that it may be freely removed and attached.

As one example of the wire stopper 24, one may cite an approximately tubular member whose outer periphery is partially notched, and whose cross-section is approximately C-shaped, but one is not limited thereto. Any type of member is acceptable so long as it is able to uniformly maintain the positional relation of the wire 5 and second sheath 6, e.g., a clip capable of attachment to the wire 5. With respect to the suture device 1 of the present embodiment, when the wire stopper 24 is interposed, the contact member 10 and distal end 6 A of the second sheath 6 are in contact.

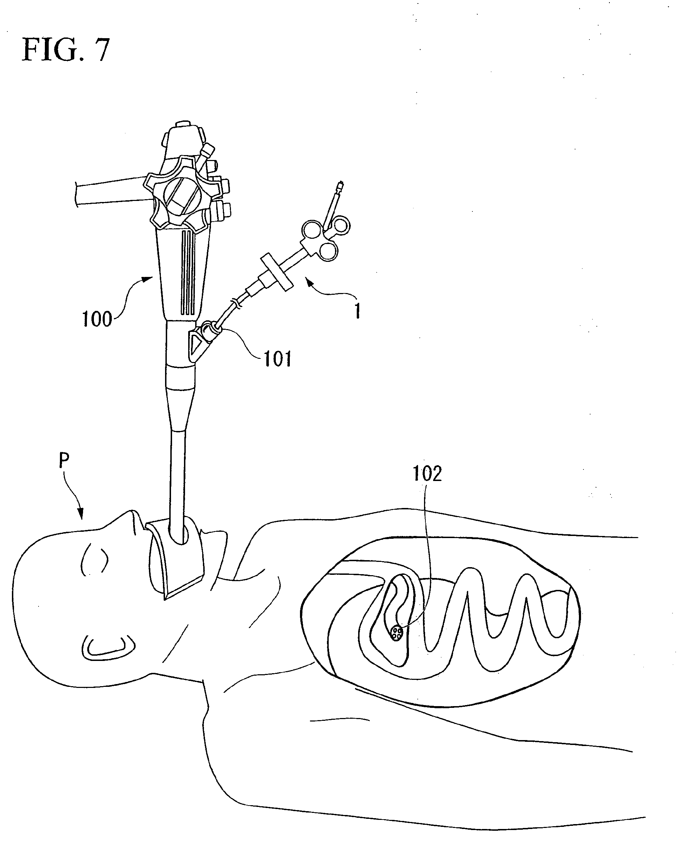

Operations during use of the suture device 1 configured in the foregoing manner are described with reference to FIG. 7 to FIG. 13.

First, as shown in FIG. 7, an endoscope 100 is inserted into the body of a patient P, and the distal end portion of the endoscope 100 is moved near the tissue which is the treatment target such as a perforation.

Next, the distal end portion of the suture device 1 is inserted into a forceps port 101 of the endoscope 100, and the distal end portion 2 of the suture device 1 is exposed from an operating channel 102.

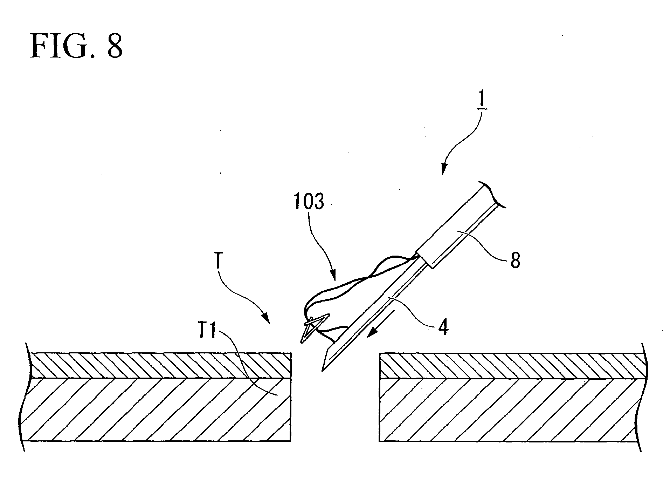

The user slides the slider 18 forward. Thereupon, as shown in FIG. 8, the needle 4 and the suture unit 103 attached to the needle 4 are exposed from the distal end portion of the first sheath 8. At this time, the projection amount of the needle 4 from the first sheath 8 may be adjusted as necessary by adjusting the fixed position of the adjuster 16 relative to the body 12 and by causing the slider 18 to contact the adjuster 16.

When the slider 18 slides forward, the distal-end operating portion 14 also slides forward together therewith, with the result that there is no change in the relative positional relations of the tube 7 and needle 4, and of the wire 5 and second sheath 6.

As shown in FIG. 8, the user causes the distal end of the suture device 1 to approach the target tissue T around the perforation or the like in a state where the needle 4 is projecting, and causes the needle 4 to cut into and pierce tissue T1 on one side.

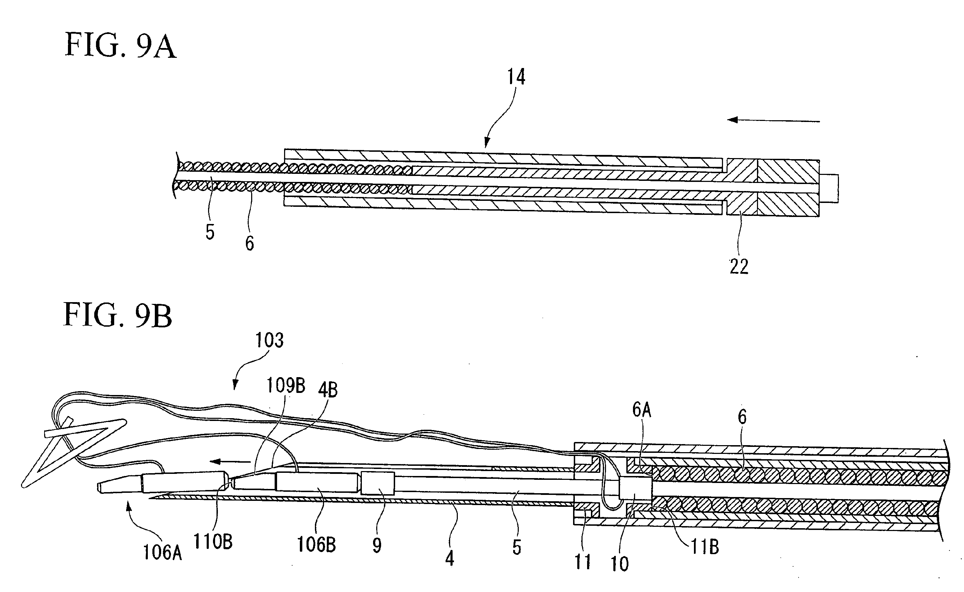

When the needle 4 has pierced it, as shown in FIG. 9A, the user pushes in the sheath operating member 22 of the distal-end operating portion 14 toward the front. Thereupon, the second sheath 6 slides forward. At this time, as shown in FIG. 9B, as the distal end 6A of the second sheath 6 contacts the contact member 10 of the wire 5, the wire 5 is pressed by the second sheath 6 and moves forward together with the second sheath 6 while maintaining the fixed relative positional relation.

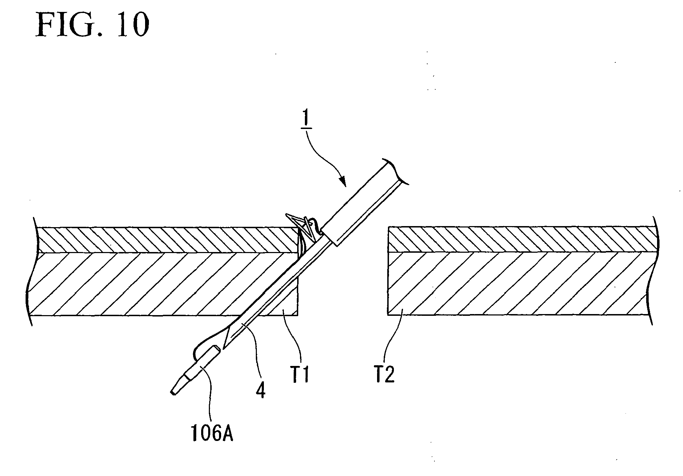

The user pushes in the sheath operating member 22 until the distal end 6A of the second sheath 6 contacts the rear end 11B of the connecting tube 11, thereby regulating the forward movement of the second sheath 6. Thereupon, as shown in FIG. 9B and FIG. 10, the first anchor 106A and second anchor 106B of the suture unit 103 move forward pressed by the pressure member 9 at the distal end of the wire 5, and the first anchor 106A is ejected to the exterior of the needle 4. The user is able to perceive that the first anchor 106A has been ejected by sensing the contact of the second sheath 6 and connecting tube 11.

At this time, as shown in FIG. 9B, the rear end of the first anchor 106A is fully pushed out to the front by the distal end face 110B of the second anchor 106B, with the result that the first anchor 106A is smoothly ejected without interfering with the needle 4. As the sloped face 109B is formed on the second anchor 106B, even when the first anchor 106A is fully pushed out, the second anchor 106B does not project from the end face 4B of the needle 4.

The user withdraws the needle 4 from the tissue T1. At this time, the first anchor 106A engages with the tissue T1. The needle 4 is then inserted into tissue T2 opposite tissue T1 sandwiching the perforation or the like, and pierces it. As the second anchor 106B does not project from the end face 4B of the needle 4, the second anchor 106B does not constitute an impediment when inserting the needle 4.

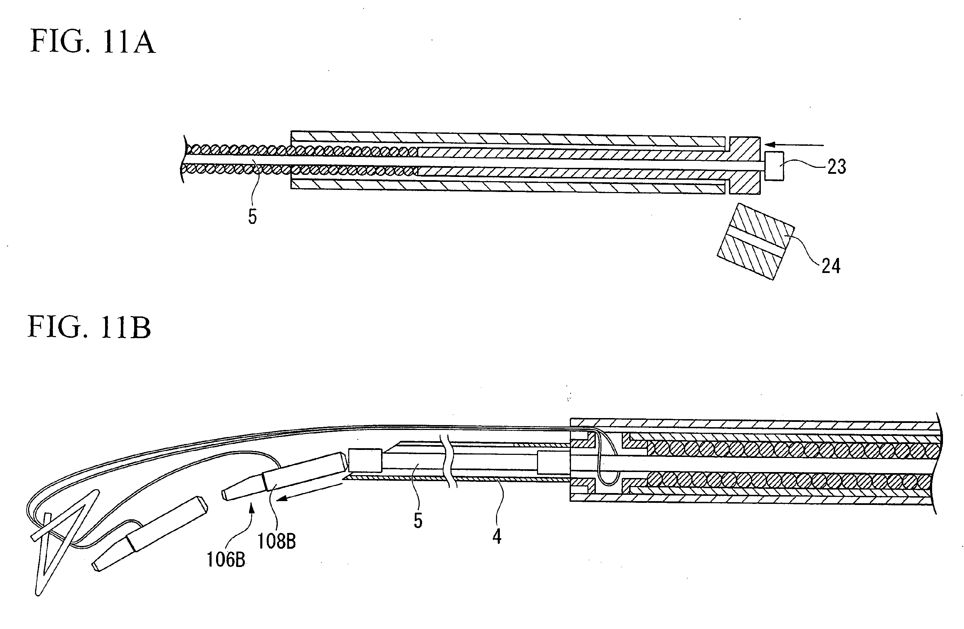

After the needle 4 has pierced the tissue T2, as shown in FIG 11A, the user removes the wire stopper 24, manipulates the wire operating knob 23, and pushes forward the wire 5. Thereupon, as shown in FIG. 11B, the wire 5 moves further forward, and the second anchor 106B is ejected to the exterior of the needle 4. As the large-diameter portion 108B of the second anchor 106B generates a clicking sensation when it passes over the projection 4C (not illustrated) of the needle 4, the user is able to easily perceive the timing of the ejection of the second anchor 106B.

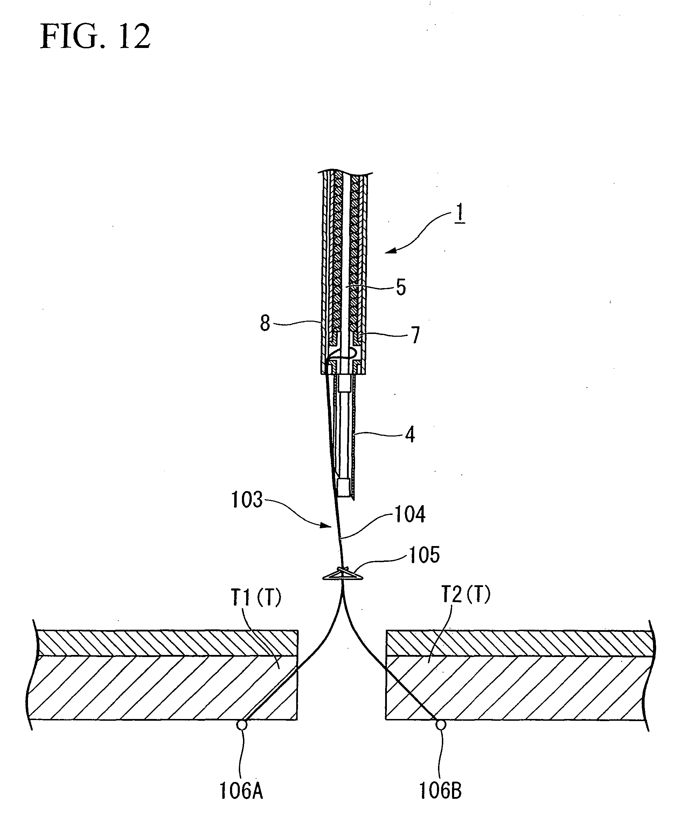

After ejection of the second anchor 106B, as shown in FIG. 12, the user withdraws the needle 4 from the tissue T2, and the second anchor 106B engages with the tissue T2.

In this state, the user pulls the slider 18 toward the proximal end 12B side of the body 12, and the needle 4 and tube 7 are housed into the first sheath 8. At this time, as the distal-end operating portion 14 also moves back together with the slider 18, the wire 5 also moves back.

Thereupon, the suture thread 104 of the suture unit 103 which is wrapped around the wire 5 is also received into the first sheath 8, and the stopper 105 and distal end of the first sheath 8 come into contact. When the user causes the slider 18 to move back further, the suture thread 104 alone is received into the second sheath 6 while the stopper 105 and second sheath 6 are in contact, thereby shortening the distance between the stopper 105 and each anchor 106Aand 106B.

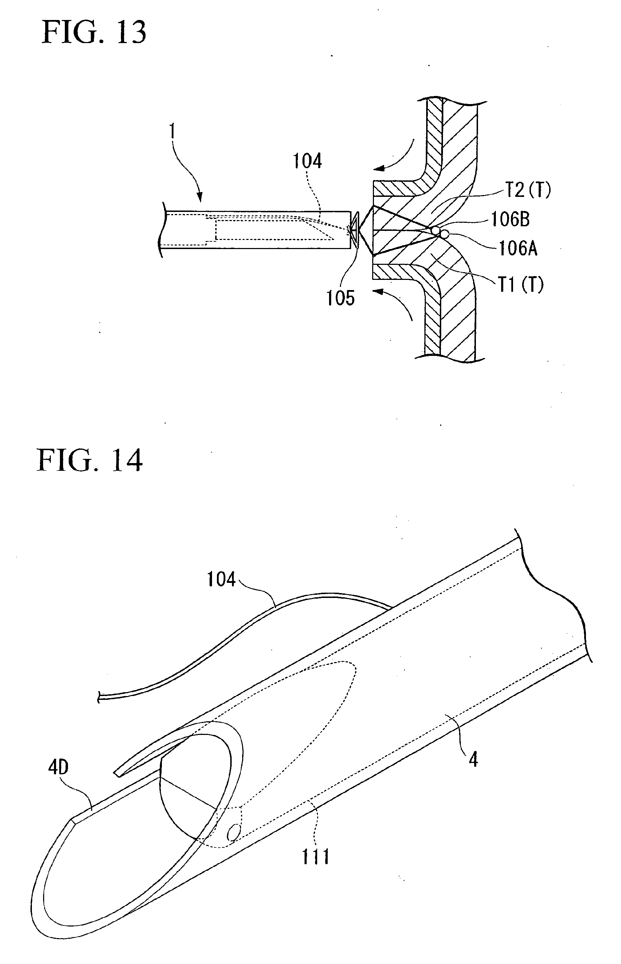

As each anchor 106A and 106B is respectively engaged with tissues T1 and T2, as shown in FIG. 13, as the stopper 105 and each anchor 106A and 106B come closer together, the tissues T1 and T2 are pulled together and sealed on the suture device 1 side along with each anchor 106A and 106B. In this manner, suturing of the target tissue T is accomplished.

At this tune, the engagement of the end portion 105A and end portion 105B of the stopper 105 (see FIG. 3) loosens when the suture thread 104 moves toward the center point 104B side and is received into the first sheath 8, but even if the suture thread 104 undertakes to move toward the side of each anchor 106A and 106B, it is unable to move in the pertinent direction, because the end portion 105A and end portion 105B engage more firmly due to the force exerted upon the suture thread 104. That is, as the stopper 105 moves only toward the side of each anchor 106A and 106B, and as it does not move toward the opposite side, loosening or dissolution of the suture of the target tissue T does not occur.

After termination of suturing, the user pulls the wire operating knob 23, and withdraws the wire 5 from the tube 7. When the distal end of the wire 5 has moved more to the rear than the connecting tube 11, the suture thread 104 is removed from the wire 5, and the suture unit 103 is severed from the suture device 1. In this manner, the series of operational steps is brought to a close.

According to the suture device 1 of the present embodiment, as the sloped face 109B is provided on the second anchor 106B, even if the first anchor 106A is fully pushed out to an extent where it does not catch on the needle 4, the second anchor 106B does not protrude from the end face 4B of the needle 4. Accordingly, the first anchor 106A can be smoothly ejected, and insertion of the needle 4 at the time of ejection of the second anchor 106B can be easily conducted.

In addition, as the distal end face 110B which is approximately orthogonal to the axis is formed at the distal end of the second anchor 106B, the distal end of the second anchor 106B is not pushed under the first anchor 106A, and the first anchor 106A to the front is reliably pushed out by the distal end face 110B.

Moreover, as a result of the contact of the contact member 10 of the wire 5 with the second sheath 6, it can be made to slide toward the needle 4 while the projection length of the wire 5 from the second sheath 6 is kept uniform. The first anchor 106A is then ejected from the needle 4 by causing the connecting tube 11 to contact the second sheath 6.

Even supposing that the tube 7 were to extend in the axial direction during the anchor ejection operations, as the shapes of the needle 4 and connecting tube 11 hardly change, the ingress of the wire 5 into the needle 4 would remain at a constantly fixed length if the second sheath 6 and connecting tube 11 were to come into contact. Accordingly, by having the second sheath 6 and connecting tube 11 come into contact, it is possible to reliably eject the first anchor 106A only, and to prevent malfunctions such as the mistaken simultaneous ejection of the first anchor 106A and second anchor 106B. As the suture thread 104 of the suture unit 103 is inserted from the through-hole 11 A of the connecting tube 11 and wrapped around the wire 5, it is possible to suture the target tissue by manipulating the slider 18 so that the needle 4 is received into the first sheath 8. Accordingly, it is possible to complete suture treatment with only the suture device 1, without using any other tool such as a clip.

Furthermore, as the wire stopper 24 is interposed between the sheath operating member 22 and wire operating knob 23, it is possible to prevent malfunctions such as mistaken forward movement of the wire 5 alone and simultaneous ejection of the anchors 106A and 106B when sliding the second sheath 6 forward and ejecting the first anchor.

In addition, as the wire operating knob 23 is configured in a shape which engages with the sheath operating member 22, no injury is done to tissue by the distal end of the wire 5 due to excessive forward movement of the wire 5 during ejection of the second anchor 107.

If the length of the wire 5 and tubular member 21 is set so that the second anchor 107 is ejected when the wire operating knob 23 contacts the sheath operating member 22, it is possible for the user to easily perceive that the second anchor 107 has been ejected.

In the foregoing embodiment, the case was described where the relative positional relation of the wire 5 and second sheath 6 is kept uniform by a contact member 10 provided in the wire 5, but it is also acceptable to keep the pertinent relative positional relation uniform by another method.

For example, in a state of intermediate placement of the wire stopper 24, it is also possible to reliably eject only the first anchor 106 by integrally grasping the sheath operating member 22 and wire operating knob 23 and sliding them forward, and by pushing in the first sheath until it contacts the connecting tube 11. In this case, the wire stopper 24 functions as a relative position maintenance member which maintains the relative positional relation of the wire 5 and second sheath 6 in a uniform state.

In the foregoing embodiment, the case was described where the parallel member is the second sheath 6 through which the wire 5 runs, but instead of this, it is also acceptable to configure the suture device so that a second wire - which runs side by side with the wire 5 - passes through the interior of the tube 7 as an parallel member, and a semi-circular or donut-shaped member is fixed to the distal end of the second wire to cause contact with the contact member 10, whereby the relative positional relation of the wire 5 and second wire could be uniformly maintained while enabling forward sliding.

Furthermore, in the foregoing embodiment, the case was described where the communicating portion through which suture thread 104 of the suture unit 103 is inserted is a through-hole provided in the connecting tube 11, but instead of this, it is also acceptable to provide the communicating portion in the outer circumferential face of the needle 4 or tube 7. Moreover, the communicating portion is not indispensable to the present invention, and as with the suture device recorded in International Patent Application Publication, First Publication No. 2007-37326, it is also acceptable to conduct suturing by severing the suture unit and suture device when the anchors are ejected, and with use of other equipment such as clips.

In addition, the pressure member 9 is not indispensable to the suture device of the present invention, and it is also acceptable to adopt a configuration where the anchors are directly pushed and ejected by the distal end of the wire 5. The adjuster 16 is similarly not indispensable, and may be omitted.

The above has been a description of a preferred embodiment of the present invention, but the present invention is not limited to such working examples. Additions, omissions, substitutions, and other modifications can be made to the configuration within a scope that does not depart from the intent of the present invention.

For example, in the above-described embodiment, the case was described where the suture thread 104 is connected so that the phases in the circumferential direction with the rear ends of the sloped faces 109A and 109B of each anchor 106A and 106B are approximately identical, but the connection mode of anchors and suture thread is not limited thereto. That is, it is acceptable to conduct positioning so that the end face 4A of the needle and the sloped faces of the anchors have the same orientation when the anchors are housed inside the needle 4 in a state where the connection sites of the suture thread 104 to the anchors are opposite the groove 4A and where the suture thread 104 runs straight through the groove 4A of the needle. Consequently, as hi the modified example shown in FIG. 14, it is also acceptable to provide a groove 4D at a position where the phase in the circumferential direction with the rear end of the end face 4B is different, and to connect the suture thread 104 to the outer circumferential face of an anchor 111 or the like corresponding to the groove 4D.

In this case, it is acceptable to move the connection site of the anchor 111 and suture thread 104 more toward the distal side than the rear end of the sloped face. If this is done, it is possible to increase diameter without providing the anchor 111 with a small-diameter portion. Accordingly, the superficies of the anchor 111 which contacts the tissue during engagement with tissue is enlarged, and it is possible to reduce stimulation to the patient by dispersing the force Imparted to the anchor.

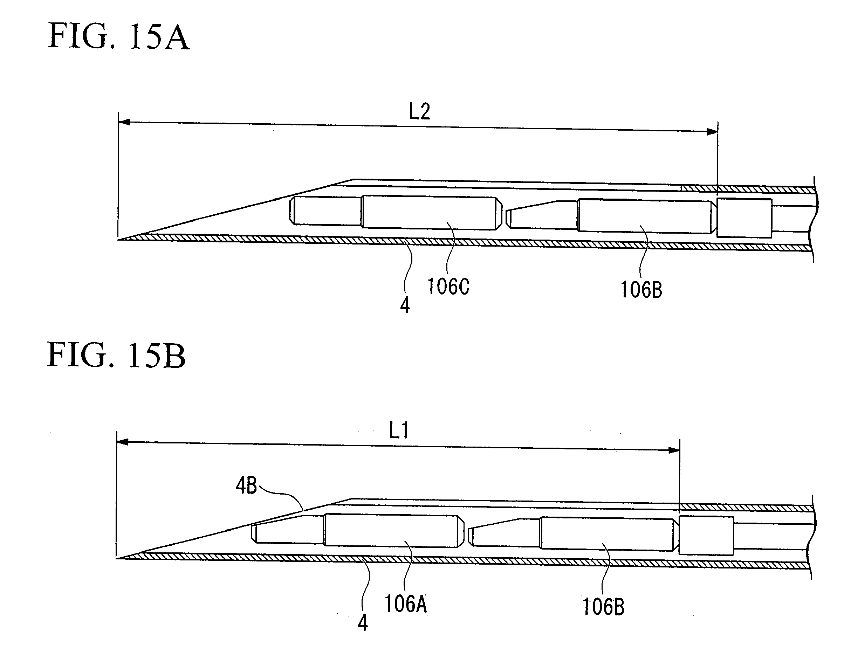

In the above-described embodiment, the case was described where both anchors are provided with sloped faces, but as it is sufficient if the first anchor 106A is reliably pushed out, it is also acceptable - as hi the modified example shown in FIG 15A - to not provide a first anchor 106C on the distal side with a sloped face, and to provide a sloped face only on the second anchor 106B housed on the proximal side.

However, when each anchor is given the same shape as in the present embodiment, cost is reduced due to the use of common members. Furthermore, if the anchors are of the same length, it is possible to house the anchors more toward the distal side without projection from the end face 4B of the needle 4. Accordingly, as shown in FIG. 15B, as the length L1 from the distal end of the needle 4 to the rear end of the second anchor 106B is shorter than length L2 in the case of the modified example shown in FIG. 15A, it is possible to shorten the length of the needle 4, and to improve insertability of the distal end 2 of the suture device 1.

In the above-described embodiment, the case was described where the suture unit 103 has two anchors, but it is also acceptable to have a suture unit provided with three or more anchors according to the form or the like of the wound which is targeted. In this case, the same effects can be obtained if all anchors excepting the anchor housed farthest toward the distal side inside the needle 4 (i.e., all anchors from the second anchor from the distal end) are provided with sloped faces.

Furthermore, in the above-described embodiment, the case was described where the anchor is ejected and engages with tissue after the needle has pierced the tissue, but instead of this, it is also acceptable to stop the distal end of the needle inside the tissue, eject the anchor inside the tissue, and cause engagement with the surrounding tissue.

In addition, in each of the above-described embodiments, the case was described where the distal-end operating portion through which the proximal end of tne wire (and the parallel member) runs is fixed to the slider of the sliding portion, but a distal-end operating portion is not indispensable to the present invention, and it is also acceptable to have the proximal end of the wire and the like exposed as is. However, when the wire and the like run through a distal-end operating portion, one can move the tube forward and backward while maintaining the positional relation of the tube and wire, thereby enabling marked improvement in operability.

Otherwise, the present invention is not limited by the foregoing description, and is only limited by the scope of the appended claims.

## Claims

1. A suture device (1) which sutures tissue using a suture unit having a first anchor (106A, 106C) and a second anchor (106B) respectively attached to the end portions of a suture thread(104) ,
wherein a hollow distal member (4) is provided which houses said first anchor and second anchor,
the end face of the distal side of said distal member is formed so as to constitute a first sharp angle with the axis of said distal member,
the distal side of said second anchor is provided with a sloped face (109B) so as to constitute a sharp angle with the axis of said second anchor which is an angle at or below said first sharp angle,
and said first anchor and said second anchor are axially aligned and housed inside said distal member so that said second anchor is positioned on the proximal side.

2. The suture device according to claim 1, wherein a distal end face (110B) which is orthogonal to the axis of said second anchor is formed on the distal side of said sloped face of said second anchor.

3. The suture device according to claim 1, wherein a sloped face (109A) is provided on the distal side of said first anchor so as to constitute a sharp angle with the axis of said first anchor which is an angle at or below said first angle.

4. The suture device according to claim 1, wherein said distal member has a groove (4A) through which said suture thread runs when said first anchor and said second anchor are housed,
and said end face of said distal member and said sloped face of said second anchor have the identical orientation when said second anchor is housed inside said distal member so that the connection site of said suture thread to the second anchor is opposite to said groove.
